# EUROPÄISCHE PATENTSCHRIFT

(11) **EP 1 143 938 B1**
(45) Veröffentlichungstag und Bekanntmachung des Hinweises auf die Patenterteilung: **18.07.2007**
(21) Anmeldenummer: 00901097.6
(22) Anmeldetag: 13.01.2000
(51) Int. Cl.: A61K 9/70, A61K 31/485

(54) **TRANSDERMALES THERAPEUTISCHES SYSTEM MIT EINER SELBSTKLEBENDEN MATRIX, ENTHALTEND ORGANISCHE SÄURE-ADDITIONSSALZE VON ALKALOIDEN DES MORPHIN- BZW. MORPHINANTYPS**
TRANSDERMAL THERAPEUTIC SYSTEM WITH SELF-ADHESIVE MATRIX, CONTAINING ORGANIC ACID ADDITION SALTS OF MORPHINE- OR MORPHINAN-TYPE ALKALOIDS
SYSTEME THERAPEUTIQUE TRANSDERMIQUE A MATRICE AUTOCOLLANTE CONTENANT DES SELS D'ADDITION D'ACIDE ORGANIQUE D'ALCALOIDES DU TYPE MORPHINE OU MORPHINANE

(30) Priorität: 14.01.1999 DE 19901085
(43) Veröffentlichungstag der Anmeldung: 17.10.2001
(73) Patentinhaber: LTS LOHMANN Therapie-Systeme AG, 56626 Andernach (DE)
(72) Erfinder: KOCH, Andreas, D-56581 Melsbach (DE); ADAM, Bernd, D-34613 Treysa (DE); MATUSCH, Rudolf, D-35041 Marburg (DE); ASMUSSEN, Bodo, D-56170 Bendorf-Sayn (DE)
(74) Vertreter: Flaccus, Rolf-Dieter
(86) Internationale Anmeldenummer: PCT/EP2000/000198
(87) Internationale Veröffentlichungsnummer: WO 2000/041683

(56) Entgegenhaltungen:
- EP-A- 0 430 019
- EP-A- 0 617 972
- WO-A-91/19474
- WO-A-97/39742

## Beschreibung

Die Erfindung betrifft ein transdermales therapeutisches System (TTS), umfassend eine wirkstoffundurchlässige Trägerschicht, eine selbstklebende Matrix aus einem kationischen Grundpolymer, bei dem es sich um ein wasserlösliches Polyaminomethacrylat handelt, und eine ablösbare Schutzfolie auf der hautzugewandten Seite, wobei die Matrix als Wirkstoff organische Säureadditionssalze von Morphin-Alkaloiden auf Basis eines fettsäurehaltigen, wasserlöslichen Polyaminomethacrylatsalzes enthält, dadurch gekennzeichnet, dass dem kationischen Grundpolymer 0,1 bis 50 Gew.-%, bezogen auf das Gesamtgewicht der Polymermischung, eines nicht wasserlöslichen bzw. vergleichsweise gering wasserlöslichen, bis zu 15 Gew.-% ternäre Dimethylaminoethyl-Gruppen enthaltenden Polyaminomethacrylats mit nicht vollständiger Quarternierung der Dimethylaminoethyl-Gruppen beigemischt ist, bei dem es sich um ein Copolymerisat aus Acryl- und Methacrylsäureestern handelt, wobei die Polymethacrylate als Mischung von quartären Ammoniumsalzen mit unterschiedlichem Versalzungsgrad und unterschiedlicher Permeabilität vorliegen, und das TTS 0,1 bis 15 Gew.-% Ölsäure und/oder Polyoxyethylensorbitanfettsäureester, beispielsweise Tween 20, oder Polyoxyethylenalkohole, beispielsweise Polymerisationsprodukte von bis zu 10 Molekülen Ethylenoxid mit je einem Molekül Octanol, Decanol oder Dodecanol bzw. Mischungen dieser Polymerisationsprodukte als Penetrationsverstärker enthält.

Die vorgenannten Wirkstoffe, enthaltend organische Säureadditionssalze von Morphin-Alkaloiden, werden bekanntlich zur Schmerzbehandlung starker bis stärkster Schmerzen oder zur Behandlung bei Drogenmißbrauch eingesetzt, wobei das transdermale therapeutische System hohen Anforderung bezüglich Hautflux, Klebkraft und Scherfestigkeit genügen muß.

Gegenüber herkömmlichen Arzneiformen zur oralen oder parenteralen Applikation bieten bekanntlich transdermale therapeutische Systeme (TTS) unter anderem den Vorteil eines steuerbaren Plasmaspiegels im therapeutischen Bereich bei gleichzeitiger Vermeidung von Unter- oder Überschreitung, der therapeutischen Dosis, was insbesondere bei starken Narkotika wie Morphin zu suchterzeugenden Effekten führen könnte.
Ein weiterer Vorteil von TTS liegt in der Umgehung des Magen-Darm-Traktes. Dadurch kann auf eine hohe Arzneistoffdosis, die dem First-pass-Effekt Rechnung zu tragen hat und für nebenwirkungsbelastete Plasmapeaks verantwortlich ist, verzichtet werden.

Für die Indikation einer Behandlung bei Drogenmißbrauch kommt als weiterer Vorteil die Sicherheit bezüglich Mißbrauch hinzu. Die Therapie kann auch von Patienten ohne unmittelbare Beaufsichtigung bzw. Kontrolle durchgeführt werden, weil eine mißbräuchliche Anwendung deshalb nicht möglich ist, weil eine Extraktion bzw. Isolierung des Wirkstoffes aus der TTS-Matrix ohne fachmännische Kenntnisse und Hilfsmittel nicht möglich ist.

Um therapeutisch wirksame Plasmaspiegel zur Behandlung schwerster Schmerzzustände zu erreichen, sind bei der transdermalen Therapie mit Morphin-Alkaloiden äußerst hohe Abgabe- bzw. Permeationsraten erforderlich, beispielsweise 2,5 bis 10 mg pro Tag.

DE 197 42 296 berichtet über eine überraschende Feststellung, wonach ausgewählte Säureadditionssalze von Morphin-Alkaloiden gegenüber den freien Basen eine wesentlich erhöhte Permeationsrate besitzen, welche als reale Hinweise auf eine günstige Erfolgsaussicht für die praktische Anwendung dieser Salze mit den geforderten hohen Permeationsraten zu werten sind.

WO 98/09591 beschreibt den Aufbau eines TTS auf Basis eines Acrylat- und Polyisobutylen-Klebers für Salze aus verschiedenen Wirkungsklassen, jedoch keine aus der Wirkungsklasse der Opiate.

EP 0 415 055 B1 beschreibt selbstklebende, hydrophile Polyaminomethacrylate, die durch das Vorliegen als Polymersalze bei vollständiger Quarternierung der Dimethylaminoethyl-Gruppen durch Fettsäuren eine vergleichsweise hohe Wasserlöslichkeit besitzen.
Infolge der guten Wasseraufnahmefähigkeit führt diese zum Quellen der Matrix und damit zur Unterbrechung der Kontaktzone Haut/Klebermatrix, wobei auch die Scherfestigkeit dieses Systems verringert wird, wodurch sowohl technische Fertigungsprobleme als auch eine zu geringe Haftfähigkeit vorprogrammiert sind. Für eine praktische Verwendung als TTS-Matrix sind daher selbstklebende, hydrophile Polyaminomethacrylate der vorgenannten Art nicht geeignet. Durch die hohe Affinität zu hydrophilen Wirkstoffen wie beispielsweise Salzen, wird zudem passive Diffusion gebremst, was sich negativ auf den Hautflux auswirkt. Verstärkt wird dieser Nachteil durch die Wasseraufnahme, weil sie die thermodynamische Aktivität des hydrophilen Wirkstoffes in der hydrophilen Matrix wesentlich reduziert.

DE 197 42 296 beschreibt Säureadditionssalze von Morphinalkaloiden in einer allgemeinen TTS-Formulierung, eine spezielle TTS-Matrix-Zusammensetzung ist nicht erwähnt. Auf eine Matrix auf Basis eines fettsäurehaltigen, wasserlöslichen Polyaminomethacrylatsalzes findet sich kein Hinweis.

EP-A-617972 offenbart ein dermales therapeutisches System aus einer schmelzfähigen Poly(meth)-acrylat Mischung.

WO-A-9739742 offenbart ein transdermales Verabreichungssystem enthaltend Dextromethorphan in einer Poly(meth)-acrylat Mischung.

Aus dem vorgenannten Stand der Technik ist zu folgern, daß die galenische Umsetzung für diesen Typ von Salzen als Wirkstoffe in einem transdermalen therapeutischen System mit selbstklebender Matrix eine spezielle Auswahl bestimmter Kombinationen medizinischer Haftkleber mit geeigneten Hilfsstoffen erfordern, die sich von selbstklebenden druckempfindlichen Reservoirsysteme kovalenter Arzneistoffe deutlich unterscheiden, wobei ein solches Matrixsystem zu bestimmende Anforderungen bezüglich Hautflux, Klebkraft und Scherfestigkeit zu erfüllen hat.
Ausgehend hiervon liegt der Erfindung die Aufgabe zugrunde, das kationische Grundpolymer so mit einem geeigneten Hilfsstoff wie Folienbildner, Weichmacher, Copolymer aufzubereiten, daß die folgenden Eigenschaften der selbstklebenden Matrix erreicht werden:
- Verringerung der Wasseraufnahme bzw. Wasserlöslichkeit,
- Erhöhung der Scherfestigkeit,
- Erhöhung des Diffusionskoeffizienten des hydrophilen Wirkstoffes.

Die Lösung der Aufgabe gelingt in überraschender Weise durch ein TTS mit den Merkmalen des Hauptanspruchs, bei dem es sich um eine Kombination des wasserlöslichen Polyaminomethacrylates als Grundpolymer für die Matrix mit einem nicht wasserlöslichen bzw. vergleichsweise gering wasserlöslichen, bis zu 15 Gew.% ternäre Dimethylaminoethyl-Gruppen enthaltenden Polyaminomethacrylat zu einer homogenen, einschichtigen Matrix handelt, wobei die Polymethacrylate als Mischung von quartären Ammoniumsalzen mit unterschiedlichem Versalzungsgrad und unterschiedlicher Permeabilität vorliegen.
Eine bevorzugte Lösung der Aufgabe besteht somit in der Maßnahme, daß dem kationischen Grundpolymer ein begrenzt wasserlösliches Copolymerisat aus Acryl- und Methacrylsäureestern mit einem vergleichsweise geringen Gehalt an quartären Ammoniumgruppen, die mit anorganischen Säuren ein Salz bilden, beigemischt ist.

Dabei befindet sich der Wirkstoff im gelösten Zustand in einer selbstklebenden, einschichtigen Matrix, die gleichzeitig für den intensiven Hautkontakt und die Haftung auf der Haut geeignet ist.

Eine Ausgestaltung des TTS nach Anspruch 1 sieht erfindungsgemäß vor, daß das Grundpolymer 0,1 bis 50 Gew.% des zugesetzten Copolymerisates mit nicht vollständiger Quarternierung der Dimethylaminoethyl-Gruppen enthält.
Eine bevorzugte Ausgestaltung des TTS sieht vor, daß das Grundpolymer bevorzugt 3 bis 30 Gew.% des zugesetzten Copolymerisates mit nichtvollständiger Quarternierung der Dimethylaminoethyl-Gruppen enthält; schließlich hat sich als besonders bevorzugt herausgestellt, daß das Grundpolymer der Matrix 10 bis 25 Gew.% des zugesetzten Copolymerisates mit nichtvollständiger Quarternierung der Dimethylaminoethyl-Gruppen enthält.
Wesentliche Verbesserungen der erwünschten Permeationsrate zur Erzielung eines therapeutisch wirksamen Plasmaspiegels bei der Behandlung schwerster Schmerzzustände durch intensivierten Hautflux des Wirkstoffs durch die Haut in den Organismus wird erfindungsgemäß dadurch erreicht, daß das TTS 0,1 bis 15 Gew.% Penetrationsverstärker enthält. Diese können der Polymermatrix gelöst eingearbeitet sein. Unter den Penetrationsverstärkern sind Polyethylensorbitanfettsäureester wie beispielsweise Tween 20 oder Polyoxyethylenalkohole wie z.B. Polymerisationsprodukte von bis zu 10 Molekülen Ethylenoxid mit je einem Molekül Octanol, Decanol oder Dodecanol oder Mischungen dieser Polymerisationsprodukte sowie Ölsäure mit Vorteil einzusetzen.

Die Konzentration des Wirkstoffes im TTS hängt mindestens teilweise von der Größe der Kontaktfläche des Reservoirs ab. Die Konzentration kann zwischen 0,1 und 50 Gew.% an organischem Morphin-Alkaloidsalz als Wirkstoff enthalten. Bevorzugt ist ein Gehalt zwischen 3 und 25 Gew.%, insbesondere hat sich eine Konzentration des Wirkstoffs zwischen 10 und 20 Gew.% des organischen Morphin-Alkaloidsalzes für den Großteil der medizinischen Indikationen vorteilhaft bewährt.

Eine Ausgestaltung des TTS nach der Erfindung sieht weiter vor, daß das Alkaloid vom Morphin- bzw. Morphinantyp Morphin, Codein, Diamorphin, Ethylmorphin, Levorphanol (Morphinantyp), Hydromorphon, Oxycodon, Oxymorphon, Hydrocodon, Dihydrocodein, Pholcodin, Nalbuphin oder Buprenorphin ist. Bei einer vorteilhaften Ausführungsform der Erfindung kann die Bildung des organischen Morphin-Alkaloidsalzes durch Einbringen von Alkaloidbase und der jeweiligen Carbonsäure im stöchiometrischen Verhältnis in der Haftmatrix erfolgen.

Das TTS nach der Erfindung kann mit einem selbstklebenden Überpflaster, bevorzugt aus textilem Gewebe, zwecks zusätzlicher Fixierung auf der Haut ausgerüstet sein, wobei das Überpflaster und die Matrix mit der Träger- und Schutzfolie einen festen Verbund bilden.

Schließlich besteht die Verwendung des TTS gemäß Erfindung in der Bekämpfung hoher und extrem hoher Schmerzzustände oder in der Behandlung bei Drogenmißbrauch.

Das TTS entspricht im übrigen in seinem Aufbau bekannten Ausbildungen transdermaler therapeutischer Systeme beispielsweise durch einen festen Verbund einer Matrix mit einer Rückschicht als Trägerfolie und einer vor Applikation ablösbaren Schutzschicht, wobei diese Rückschicht undurchlässig für Wasserdampf und insbesondere für Wirkstoff sein muß und üblicherweise aus Polyester, Polypropylen oder beschichtetem Papier oder Aluminiumfolie bestehen kann. Bei einer bevorzugten Ausführungsform besteht die Trägerschicht aus Polyethylenterephthalat (PET) mit einer Schichtdicke von 15 µm, wobei das PET klar, opak oder bedruckt vorliegen kann. Weiterhin kann das TTS die übliche Ausbildung mit einer ablösbaren Schutzschicht mit einer Schichtdicke von beispielsweise 40 bis 100 µm besitzen. Diese ablösbare Schutzschicht, welche mit der Reservoirschicht in Berührung steht und vor Gebrauch vom TTS abgezogen werden muß, kann beispielsweise aus denselben Materialien bestehen, die zur Herstellung der Rückschicht bzw. Trägerfolie verwendet werden.
Die Ablösbarkeit wird beispielsweise durch Silikonbehandlung der Folienoberflächen erreicht. Auch kann die Schutzschicht neben der Silikonisierung zusätzlich noch metallisiert sein, z.B. durch Aluminiumbedampfung. Die Schutzschicht kann zusätzlich mit Applikationshilfen versehen sein, wodurch sie leichter vom TTS abgezogen werden kann. Bei einer bevorzugten Ausführungsform besteht die Schutzschicht aus Polyethylenterephthalat (PET) mit einer Schichtdicke von 100 µm, wobei das PET ebenfalls klar, opak oder bedruckt vorliegen kann.

Das folgende Beispiel beschreibt die Herstellung eines erfindungsgemäßen TTS:

6,017 g einer 52,5 %igen (Gew.%) Lösung des Polymersalzes, beispielsweise E 35 H der Firma Röhm, Darmstadt, in Ethanol, werden zusammen mit 1,661 g einer 30,1 %igen (Gew.%) Lösung des Folienbildners RL 100 der Firma Röhm, Darmstadt, in einem abgeschlossenen Rührgefäß vorgelegt. In dieser Haftkleberlösung werden unter Rühren bei Raumtemperaturen 0,500 g Ölsäure und anschließend 0,841 g Morphinium-Nikotinat jeweils portionsweise eingetragen. Nach dem Ende der Wirkstoff- und Enhancerzugabe wurde noch 30 min bei Raumtemperatur bis zur vollständigen Auflösung gerührt. Die erhaltene Lösung wurde zwecks Entgasung von Luftbläschen noch 30 min lang bei 45 °C mit Ultraschall behandelt.
Der klare und homogene TTS-Masse-Ansatz wurde anschließend mit einem Auftragegerät, z.B. einer Rakel auf eine durch Silikonbehandlung ablösbar gemachte Polyesterfolie von 100 µm Dicke, z.B. Hostaphan RN 100 der Firma Hoechst, Frankfurt, in einer Strichstärke (Naßschichtdicke) von 300 µm aufgetragen.
Nachdem die Lösemittel der Kleberlösung durch 25 minütiges Trocknen im Trockenschrank bei 50 °C entfernt wurden, erfolgte die Abdeckung des Haftkleberfilmes mit einer 15 µm dicken Polyesterfolie, z.B. Hostaphan RN 15 der Firma Hoechst, Frankfurt, durch Kaschieren.
Das resultierende Flächengewicht der Matrix betrug 273 g/m², wobei die jeweiligen Matrix-Bestandteile zu folgenden Gewichtsprozenten eingearbeitet waren:

| | |
|---|---|
| 63,18 % E 35 H - | wasserlösliches Polyaminomethacrylatsalz |
| 10,00 % RL 100 | (begrenzt wasserlösliches Copolymerisat aus Acryl- und Methacrylsäureestern mit einem geringen Gehalt an quartären Ammoniumgruppen, die mit anorganischen Säuren versalzt sind; in USP/NF als "Ammonio Methacrylate Copolymer, Typ A" beschrieben) |
| 16,82 % Wirkstoff | |
| 10,00 % Ölsäure | |

Mit Schneidewerkzeugen wurden aus den Laminaten 20 cm² große TTS ausgestanzt und jeweils in Vierrandsiegelbeuteln aus Aluminium/Polyethylen-Verbundfolie verpackt.

Weiter Einzelheiten, Merkmale und Vorteile der Erfindung ergeben sich aus der nachstehenden Erläuterung eines in den Zeichnungen schematisch dargestellten Ausführungsbeispieles. Es zeigen:
- FIG:1:: den schematischen Aufbau des Matrix-Systemes,

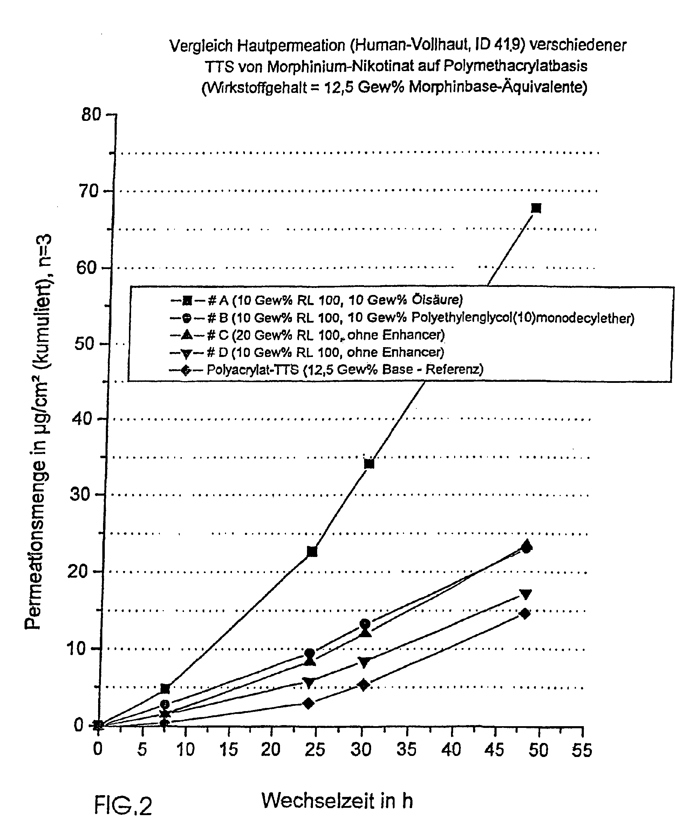

- FIG.2:: Diagramme des Permeationsverhaltens erfindungsgemäßer Formulierungen unter in-vitro-Bedingungen bei 37 °C am Hautmodell Humanhaut.

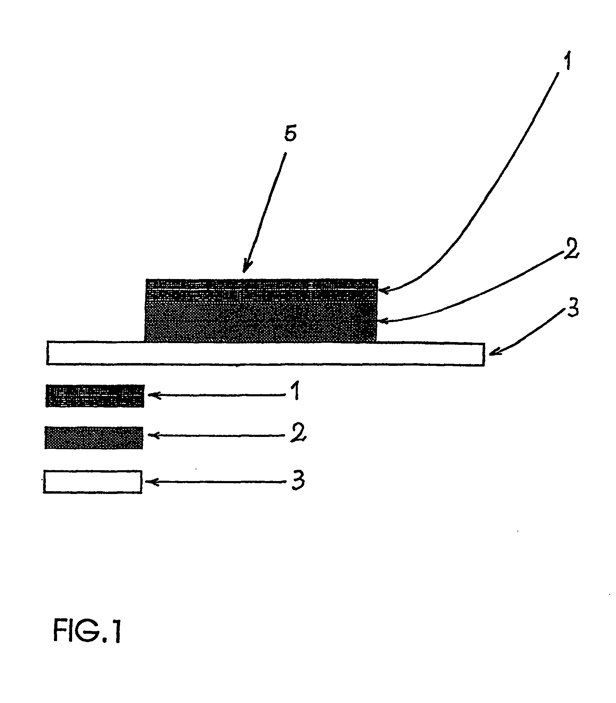

Das in FIG.1 dargestellte erfindungsgemäße TTS besteht aus einer wirkstoffundurchlässigen Trägerschicht 1, einer selbstklebenden Polymethacrylatmatrix mit Wirkstoff 2 sowie einer abzulösenden Schutzfolie 3.

FIG.2 zeigt Vergleiche der Hautpermeation an Human-Vollhaut mit verschiedenen TTS von Morphinium-Nikotinat auf Polymethacrylatbasis und einem Wirkstoffgehalt von 12,5 Gew.% Morphinbase-Äquivalent.
Als Basisvergleich dient ein Polyacrylat-TTS ohne Enhancer, wogegen die höchste Permeationsrate mit 10 Gew.% RL 100 und 10 Gew.% Ölsäure erzielt werden. Dabei ergibt sich beispielsweise eine Steigerung der Permeabilität im Verhältnis 1:4,66.
Hierbei wurden zum Vergleich TTS mit einer Permeationsfläche von 1,54 cm² ausgestanzt, auf die Hautoberfläche von präparierten Human-Vollhaut-Proben aufgeklebt und in modifizierten Franz-Diffusionszellen bezüglich ihrer Permeationsraten nach Maßgabe der Abhängigkeit der Konzentration von permeiertem Wirkstoff als Zeitfunktion im Akzeptormedium mittels HPLC untersucht. Als Akzeptor diente 0,9 %ige Kochsalzlösung mit 0,1 % Zusatz von Natriumazid.

Das erfindungsgemäße Matrixsystem ist vergleichsweise lipophil, nur noch in geringem Umfang wasserlöslich und erfüllt die mit der Aufgabenstellung genannten Eigenschaften, ohne daß die Beladungskapazität der organischen Salze vom Morphintyp sinkt. Das Matrixsystem gilt im Sinne der Erfindung als nicht vollständig wasserlöslich, wenn es in reinem Wasser bei 20 °C als 1 %ige Lösung keine klare und homogene Lösung mehr ergibt.

Die Erfindung stellt gegenüber dem Stand der Technik einen Fortschritt und eine Bereicherung im therapeutischen Bereich der Schmerzbehandlung bei starken und stärksten Schmerzen sowie bei der Behandlung von Drogenmißbrauch dar und löst in optimaler Weise die eingangs gestellte Aufgabe.

## Patentansprüche

1. Transdermales therapeutisches System (TTS), umfassend eine wirkstoffundurchlässige Trägerschicht, eine selbstklebende Matrix aus einem kationischen Grundpolymer, bei dem es sich um ein wasserlösliches Polyaminomethacrylat handelt, und eine ablösbare Schutzfolie auf der hautzugewandten Seite, wobei die Matrix als Wirkstoff organische Säureadditionssalze von Morphin-Alkaloiden auf Basis eines fettsäurehaltigen, wasserlöslichen Polyaminomethacrylatsalzes enthält, **dadurch gekennzeichnet, daß** dem kationischen Grundpolymer 0,1 bis 50 Gew.-%, bezogen auf das Gesamtgewicht der Polymermischung, eines nicht wasserlöslichen bzw. vergleichsweise gering wasserlöslichen, bis zu 15 Gew.-% ternäre Dimethylaminoethyl-Gruppen enthaltenden Polyaminomethacrylats mit nicht vollständiger Quarternierung der Dimethylaminoethyl-Gruppen beigemischt ist, bei dem es sich um ein Copolymerisat aus Acryl- und Methacrylsäureestern handelt, wobei die Polymethacrylate als Mischung von quartären Ammoniumsalzen mit unterschiedlichem Versalzungsgrad und unterschiedlicher Permeabilität vorliegen, und das TTS 0,1 bis 15 Gew.-% Ölsäure und/oder Polyoxyethylensorbitanfettsäureester, beispielsweise Tween 20, oder Polyoxyethylenalkohole, beispielsweise Polymerisationsprodukte von bis zu 10 Molekülen Ethylenoxid mit je einem Molekül Octanol, Decanol oder Dodecanol bzw. Mischungen dieser Polymerisationsprodukte als Penetrationsverstärker enthält.

2. TTS nach Anspruch 1, **dadurch gekennzeichnet, daß** dem kationischen Grundpolymer 3 bis 30 Gew.-%, besonders bevorzugt 10 bis 25 Gew.-%, bezogen auf das Gesamtgewicht der Polymermischung, des Copolymerisats aus Acryl- und Methacrylsäureestern mit nicht vollständiger Quarternierung der Dimethylaminoethylgruppen beigemischt ist.

3. TTS nach Anspruch 1, **dadurch gekennzeichnet, daß** es 5 bis 15 Gew.-%, besonders bevorzugt 10 Gew.-% Ölsäure als Penetrationsverstärker enthält.

4. TTS nach Anspruch 1, **dadurch gekennzeichnet, daß** es 5 bis 15 Gew.-%, besonders bevorzugt 10 Gew.-% Polyoxyethylensorbitanfettsäureester, beispielsweise Tween 20, oder Polyoxyethylenalkohole, beispielsweise Polymerisationsprodukte von bis zu 10 Molekülen Ethylenoxid mit je einem Molekül Octanol, Decanol oder Dodecanol bzw. Mischungen dieser Polymerisationsprodukte als Penetrationsverstärker enthält.

5. TTS nach einem der vorangehenden Ansprüche, **dadurch gekennzeichnet, daß** es 0,1 bis 50 Gew.-%, bevorzugt 3 bis 25 Gew.-% und besonders bevorzugt 10 bis 20 Gew.-%, organisches Morphin-Alkaloidsalz als Wirkstoff enthält.

6. TTS nach Anspruch 5, **dadurch gekennzeichnet, daß** die Bildung des organischen Morphin-Alkaloidsalzes durch Einbringen von Alkaloidbase und der jeweiligen Carbonsäure im stöchiometrischen Verhältnis in der Haftmatrix erfolgt.

7. TTS nach einem der vorangehenden Ansprüche, **dadurch gekennzeichnet, daß** das Alkaloid vom Morphin- bzw. Morphinantyp Morphin, Codein, Diamorphin, Ethylmorphin, Hydromorphon, Levorphanol (Morphinantyp), Pholcodin, Dihydrocodein, Hydrocodon, Oxycodon, Oxymorphon, Nalbuphin oder Buprenorphin ist.

8. TTS nach einem der vorangehenden Ansprüche, **dadurch gekennzeichnet, daß** es mit einem selbstklebenden Überpflaster, bevorzugt aus textilem Gewebe, zur zusätzlichen Fixierung auf der Haut ausgerüstet ist, wobei das Überpflaster und die Matrix mit der Träger- und Schutzfolie einen festen Verbund bilden.

## Claims

1. Transdermal therapeutic system (TTS) comprising an active substance-impermeable carrier layer, a self-adhesive matrix of a cationic base polymer, which cationic base polymer is a water-soluble polyaminomethacrylate, and a removable protective sheet on the skin-facing side, said matrix containing as active substance organic acid addition salts of morphine alkaloids based on a fatty acid-containing, water-soluble polyaminomethacrylate salt, **characterized in that** to the cationic base polymer there is admixed 0.1 to 50%-wt, relative to the total weight of the polymer mixture, of a water-insoluble or comparatively sparingly water-soluble polyaminomethacrylate containing up to 15%-wt. of ternary dimethylaminoethyl groups with incomplete quaternization of said dimethylaminoethyl groups, said polyaminomethacrylate being a copolymer of acrylic and methacrylic acid esters, the polymethacrylates being present as a mixture of quaternary ammonium salts with different degrees of salt formation and different permeabilities, and said TTS containing as penetration enhancer 0.1 to 15%-wt. oleic acid and/or polyoxyethylene sorbitan fatty acid ester(s), for example Tween 20, or polyoxyethylene alcohols, for example polymerisation products of up to 10 ethylene oxide molecules with one octanol, decanol or dodecanol molecule each, or mixtures of these polymerisation products.

2. TTS according to claim 1, **characterized in that** 3 to 30%-wt., especially preferably 10 to 25%-wt., relative to the total weight of the polymer mixture, of the copolymer of acrylic and methacrylic acid esters with incomplete quaternization of the dimethylaminoethyl groups is admixed to the cationic base polymer.

3. TTS according to claim 1, **characterized in that** it contains 5 to 15%-wt., especially preferably 10%-wt., of oleic acid as penetration enhancer.

4. TTS according to claim 1, **characterized in that** as penetration enhancer(s) it contains 5 to 15%-wt., especially preferably 10%-wt, of polyoxyethylene sorbitan fatty acid ester(s), for example Tween 20, or polyoxyethylene alcohols, for example polymerisation products of up to 10 ethylene oxide molecules with one octanol, decanol or dodecanol molecule each, or mixtures of these polymerisation products.

5. TTS according to any one of the preceding claims, **characterized in that** it contains 0.1 to 50%-wt., preferably 3 to 25%-wt., and particularly preferably 10 to 20%-wt. of organic morphine alkaloid salt as active substance.

6. TTS according to claim 5, **characterized in that** the formation of the organic morphine alkaloid salt is carried out by introducing alkaloid base and the respective carboxylic acid at a stoichiometric ratio in the adhesive matrix.

7. TTS according to any one of the preceding claims, **characterized in that** the morphine-type or morphinane-type alkaloid is morphine, codeine, diamorphine, ethylmorphine, hydromorphone, levorphanol (morphinane-type), pholcodine, dihydrocodeine, hydrocodone, oxycodone, oxymorphone, nalbuphine, or buprenorphine.

8. TTS according to any one of the preceding claims, **characterized in that** it is provided with a self-adhesive overlying patch, preferably of textile fabric, for additional fixation on the skin, said overlying patch and the matrix forming a firm composite structure with the carrier sheet and protective sheet.

## Revendications

1. Système thérapeutique transdermique (TTS) comprenant une couche de support imperméable à la substance active, une matrice autocollante d'un polymère de base cationique à propos duquel il s'agit d'un polyaminométhacrylate soluble dans l'eau, et une feuille de protection amovible sur le côté tourné vers la peau, la matrice contenant, à titre de substances actives, des sels d'addition d'acides organiques d'alcaloïdes de morphine à base d'un sel de polyaminométhacrylate soluble dans l'eau, contenant des acides gras, **caractérisé en ce que**, au polymère de base cationique, est ajouté par mélange, à concurrence de 0,1 à 50 % en poids rapportés au poids total du mélange polymère, un polyaminométhacrylate non soluble dans l'eau, respectivement relativement peu soluble dans l'eau, contenant des groupes diméthylaminoéthyle ternaires jusqu'à concurrence de 15 % en poids, les groupes diméthylaminoéthyle n'étant pas complètement quaternisés, et à propos duquel il s'agit d'un copolymère d'esters d'acide acrylique et d'acide méthacrylique, les polyméthacrylates étant présents sous la forme d'un mélange de sels d'ammonium quaternaires possédant un degré de salification différent et une perméabilité différente, et le TTS contenant, à titre de renforçateur de la pénétration, à concurrence de 0,1 à 15 % en poids, de l'acide oléique et/ou des esters d'acides gras de polyoxyéthylènesorbitanne, par exemple Tween 20, ou des polyoxyéthylènealcools, par exemple des produits de polymérisation d'un nombre de molécules d'oxyde d'éthylène allant jusqu'à 10 avec respectivement une molécule d'octanol, de décanol ou de dodécanol, respectivement des mélanges de ces produits de polymérisation.

2. TTS selon la revendication 1, **caractérisé en ce que**, au polymère de base cationique, est ajouté par mélange, à concurrence de 3 à 30 % en poids, de manière particulièrement préférée de 10 à 25 % en poids, rapportés au poids total du mélange polymère, le copolymère d'esters d'acide acrylique et d'acide méthacrylique dont les groupes diméthylaminoéthyle ne sont pas complètement quaternisés.

3. TTS selon la revendication 1, **caractérisé en ce que**, il contient, à titre de renforçateur de la pénétration, à concurrence de 5 à 15 % en poids, de manière particulièrement préférée, à concurrence de 10 % en poids, de l'acide oléique.

4. TTS selon la revendication 1, **caractérisé en ce que**, il contient, à titre de renforçateur de la pénétration, à concurrence de 5 à 15 % en poids, de manière particulièrement préférée à concurrence de 10 % en poids, des esters d'acides gras de polyoxyéthylènesorbitanne, par exemple Tween 20, ou des polyoxyéthylènealcools, par exemple des produits de polymérisation d'un nombre de molécules d'oxyde d'éthylène allant jusqu'à 10 avec respectivement une molécule d'octanol, de décanol ou de dodécanol, respectivement des mélanges de ces produits de polymérisation.

5. TTS selon l'une quelconque des revendications précédentes, **caractérisé en ce que**, il contient, à titre de substance active, à concurrence de 0,1 à 50 % en poids, de préférence de 3 à 25 % en poids et de manière particulièrement préférée de 10 à 20 % en poids, un sel organique d'alcaloïde de morphine.

6. TTS selon la revendication 5, **caractérisé en ce que**, la formation du sel organique d'alcaloïde de morphine a lieu par incorporation, dans la matrice autocollante, d'une base alcaloïde et de l'acide carboxylique respectif dans le rapport stoechiométrique.

7. TTS selon l'une quelconque des revendications précédentes, **caractérisé en ce que**, l'alcaloïde de morphine respectivement de type morphinane est la morphine, la codéine, la diamorphine, l'éthylmorphine, l'hydromorphone, le lévorphanol (type morphinane), la pholcodine, la dihydrocodéine, l'hydrocodone, l'oxycodone, l'oxymorphone, la nalbuphine ou la buprénorphine.

8. TTS selon l'une quelconque des revendications précédentes, **caractérisé en ce que**, il est équipé d'un emplâtre de revêtement autocollant, de préférence en tissu textile, pour la fixation supplémentaire sur la peau, l'emplâtre de revêtement et la matrice formant, avec la feuille de support et la feuille de protection, un composite résistant.
